# EUROPEAN PATENT APPLICATION

(11) **EP 1 743 578 A1**
(43) Date of publication of application: **17.01.2007**
(21) Application number: 06116545.2
(22) Date of filing: 04.07.2006
(51) Int. Cl.: A61B 6/00

(54) **Method and equipment arrangement for presenting information in radiology**

(30) Priority: 14.07.2005 US 181385
(71) Applicant: General Electric Company, 00510 Helsinki (FI)
(72) Inventor: Jokiniemi, Tommi, 05800 Hyvinkää (FI)
(74) Representative: LEITZINGER OY

(57) **Abstract**

An equipment arrangement in radiology, characterised in that the equipment arrangement comprises a projector to project as information relating to radiology at least image information of the object of the radiological procedure to be visible in the field of vision of a person performing the radiological procedure, which information said person utilises when performing the radiological procedure.

## Description

### Technical field

In radiology it is typical to perform invasive procedures. In these procedures accuracy is of primary importance and a desirable characteristic. An objective is also to interpret the radiological images as correctly as possible.

### Prior art

Image information obtained by radiology is utilised in performing invasive procedures. In order to obtain a sufficiently high accuracy of the invasive procedures the person performing the invasive procedures must be very focused on the performance, but still there exists a risk of an inaccurate invasive procedure.

In the following we discuss the subject with regard to mammography. For instance, the western manner of life (fatty diet etc.) has contributed to an ever more common occurrence of women's breast cancer. As this is a disease of a serious category, much attention is paid to a sufficiently early detection of breast cancer in more and more countries. Such detection is realised with mammography equipment.

For instance in Finland, every woman can get her breasts examined. A screening free of charge is made for women in a certain age group. If the screening provides any suspicious results, the examination is continued by clinical examination. In clinical examination of the observed suspicious location of the breast there is either taken additional images from different angles, or biopsy or sampling is performed at the suspicious location of the breast. In prior art the X-ray technologist taking the image draws with a pen the locations of moles, scars and other changes on a paper report. This paper report is available to the radiologist studying the images. Additional images can also be taken so that the breast is compressed or squeezed at that area where the suspicious object is located, and a magnified image is taken of the object. In this manner the aim is to obtain additional certainty about the object, and if the object still seems suspicious, then biopsy is performed. In biopsy one takes with a biopsy needle or sampling needle such an amount of cells, which is sufficient for a reliable analysis of the tissue of concern.

In prior art the sampling is made by freehand or motorised sampling. Typically two projection images are taken for freehand sampling. One projection image is taken when the breast is pressed in its lateral position by the compressing means, and the second projection image is taken when the breast is pressed in its vertical position by the compressing means. Thus the projection images are taken in a 90 degrees angle with respect to each other. On the basis of the information provided by these projection images the person taking the sample, generally a physician, directs by freehand, i.e. manually, the sample needle towards the suspicious location, which was found in the projection images and where the sampling is intended to be made. A drawback of freehand sampling or biopsy is the sampling inaccuracy, which in the worst case can lead to a false negative diagnosis.

### Short description of the invention

An object of the invention is to facilitate radiological procedures, such as manual biopsy, and to increase the accuracy of the procedures. An object of the invention is also to secure a correct interpretation of radiological images. This is attained by a method in radiology where as information relating to radiology at least image information of the object of the radiological procedure is projected to be visible in the field of vision of a person performing the radiological procedure, which information said person utilises when performing the radiological procedure.

An object of the invention is an equipment arrangement in radiology comprising a projector to project as information relating to radiology at least image information of the object of the radiological procedure to be visible in the field of vision of a person performing the radiological procedure, which information said person utilises when performing the radiological procedure.

The invention improves the ergonomics for a person performing radiological procedures, and also for the patient. The better ergonomics and the improved accuracy in performing the radiological procedures also enable an improved efficiency, which in the busy hospital environment is an important factor. In addition, the combination of the external anatomy and the internal structure provides advantages in easier interpretation of the radiological images.

An embodiment according to the invention where the image information is projected on the surface of the object is very advantageous in mammography, where the surface of a breast compressed by compression plates is even, and substantially of one colour.

### List of figures

Figure 1 shows an X-ray imaging device with an integrated biopsy device, used as a radiology equipment arrangement for mammography.
Figure 2 shows a mammography equipment arrangement as a radiology equipment arrangement according to the invention.

### Detailed description of the invention

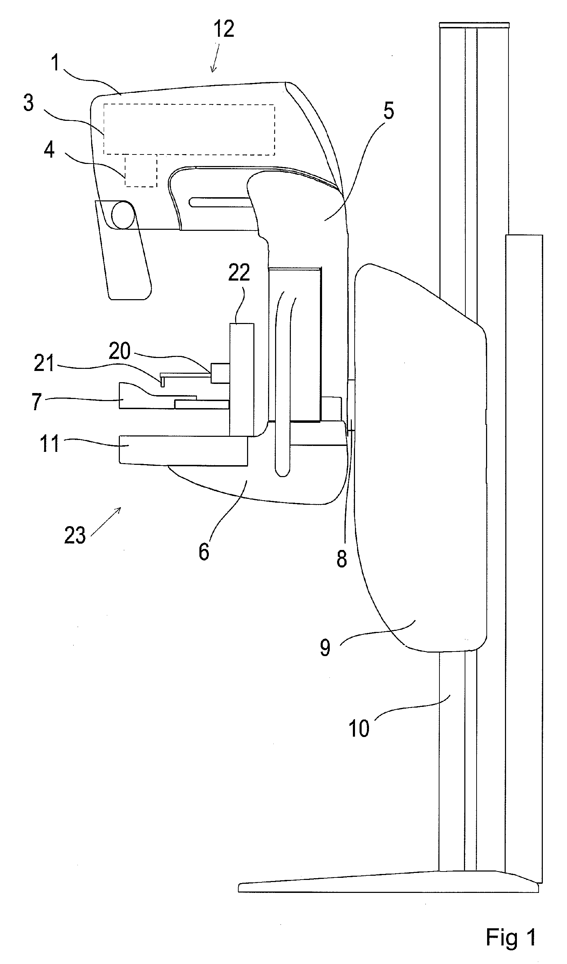

Figure 1 shows as an example of radiology equipment arrangements an X-ray imaging device used in X-ray mammography examination. The mammography device shown in figure 1 comprises a C-formed arm 1, 5, 6, in which an X-ray tube end 12 and the image generating section 23 comprising a full-field detector 11 are attached at the opposing branches 1, 6 of the console. The console further includes a movable compression plate 7 for compressing a breast. The console is mounted in bearings so that it can be turned around a shaft 8, which is attached so that it is substantially in parallel with the branches 1, 6 and substantially perpendicular to the bar 5 connecting the branches. The shaft 8 is also attached to a sliding body 9 movable along a vertical post 10, which provides the height adjustment of the device.

In the first phase of breast examination an image is taken of the whole breast. The devices in current use are to an ever-increasing degree digital mammography devices, where the X-rays radiated through the breast are received by a digital full-field detector. An example of a digital mammography device is shown in figure 1. The area of a full-field detector is generally sufficient to image the whole breast in one imaging procedure. The area of a full-field detector is for instance 18 cm x 24 cm. An X-ray image taken of the whole breast is examined using a workstation for diagnostic study, whereby the workstation typically comprises display devices with high resolution and contrast. A suspicious spot found in the X-ray image is located for sampling or biopsy. In biopsy a sample is taken from the suspicious spot of the breast, and the type of the tissue in the suspicious breast spot is identified with the aid of the sample.

With the biopsy device arrangement in a mammography device, of which an example is shown in figure 1, biopsy is performed in the following manner. The biopsy device 20 can be integrated in a digital mammography device so that the biopsy can be performed using the same full-field detector, which is used also in e.g. screening imaging. The patent application US 2002156360 shows how a full-field detector is utilised both for screening imaging and biopsy. The term "can be integrated" means in this patent application that the biopsy device can be integrated to be a fixed part of the mammography device, or that the biopsy device can be integrated into and demounted from the mammography device when needed. The integration is made so that the volume space of the sample needle 21, i.e. of the sampling, can be arranged to operate at a desired location of the imaging area of the full-field detector. The volume space comprises the target being the object of examination or sampling, in mammography the breast or a part of the breast. The cross-sectional area of the sampling volume space is for instance 5 cm x 5 cm regarding the cross-sectional area in the direction of the detector's surface. Said sampling in the volume space and moving of the volume space is performed with the needle control unit 22. The electronics and software required by the operation of the needle control unit is located in the needle control unit and/or somewhere else in the X-ray imaging device, or in a part outside the X-ray imaging device.

The biopsy can also be executed with a separately integrable e.g. 5 cm * 5 cm biopsy detector and with a separately integrable biopsy device.

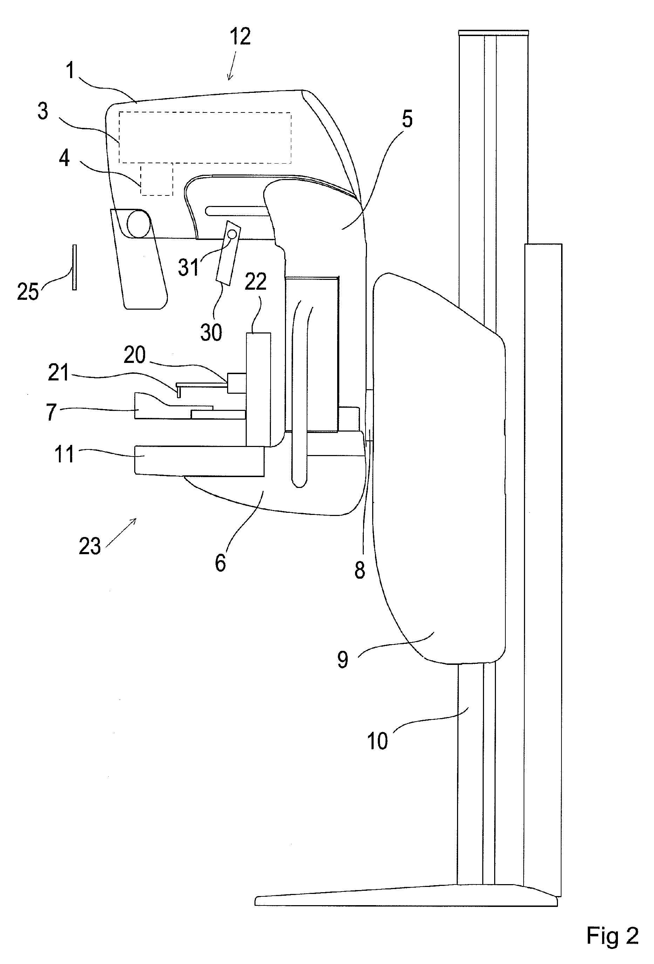

Realisation of the invention is performed for instance with the mammography equipment arrangement shown in figure 2, where the sampling is performed freehand or manually using a biopsy needle 25. In an advantageous embodiment of the invention a projector is utilised so that the projector projects X-ray image information, which models the imaging anatomy and which is taken from at least one direction, so that it is visible on the breast. In the equipment arrangement said projection can be made for instance from the effective focus via a mirror. The projector 30 shown in figure 2 is attached to the mammography device so that it can be moved at its fastening point 31. It is also possible to locate the projector separately from the mammography device.

With the projection according to the invention the image information generated by the X-ray imaging can be made visible on the surface of the breast, where one can see, among other things, the size and position of the suspicious spot. Thus the embodiment according to the invention combines both the imaging anatomy and the true anatomy on the surface of the object to be imaged. The embodiment according to the invention makes the biopsy easier, and in certain situations the accuracy in freehand biopsy is substantially increased to a success, which can be further aided by focusing at least one control mark to be visible on the surface of the breast with the aid of the projector. The control mark facilitates the positioning of an instrument used in the radiological procedure. Depending on the application the instrument can be a biopsy or sampling needle, a positioning needle, a catheter, an endoscopy instrument, or an ultrasound probe.

The correlation of visible characteristics, such as scars, moles or retractions on the external anatomy of a breast, with the X-ray information projected on the surface of the breast, provides a significant aid in the interpretation of the X-ray image information.

One utilisation method is to perform ultrasound examination of a breast, which is pressed either through a transparent trough or a hole. Visible image information aids the radiologist to position the ultrasound probe at the desired place and to better perceive the relationship between different imaging modalities, because the radiologist will continously see the position of the ultrasound probe in the X-ray image on the surface of the breast.

When utilising the projector 30 of the mammography equipment arrangement according to the invention it is possible to direct, in addition to the X-ray image information, also imaging parameters to be visible on the surface of the breast or on a part of the mammography equipment arrangement, such as the magnitude of the compression force used to compress the breast and/or the compression thickness, the voltage of the X-ray tube, the current of the X-ray tube, the filtering element of the radiation, the product of the X-ray tube current and the exposure time (mAs) and the radiation dose. This facilitates treatment, as the required information is directly visible in the field of vision of the person performing the procedure. Said part of the mammography equipment arrangement can mean for instance the imaging means or the compression means.

Particularly during invasive radiological procedures the embodiment according to the invention can utilise green coloured light for projecting both image information and numerical information on the surface of the object, which improves the ergonomics, particularly for the patients. In green coloured light the human visual organs will perceive blood running from the puncture point as dark matter, which for a human being is a much more pleasant view than red blood. The green coloured light can be created at the projector for instance with a colour projector or by a field illumination embodiment, where the illuminating light is green. In a mammography equipment arrangement, and also in other radiological equipment arrangements, also some other means may be relevant for changing the red colour of blood to a dark colour for human visual organs. As examples of such means we can mention glasses with green lenses for the patient. Correspondingly, also for instance in blue light or with glasses with blue lenses, the human visual organs will see the red colour of blood as a darker colour.

The projector 30 used according to the invention such as for example in the mammography equipment arrangement shown in figure 2, can be of the size of for instance only a mobile phone, and conveniently it can be integrated in connection with the mammography device.

We have not presented more detailed technical embodiments than those set forth above, because they can be realised in mechanics, electronics and software by embodiments according to prior art.

However, even if the invention was presented above with reference to the enclosed figures and to the description, the invention is not limited to these, but the invention can be modified within the scope permitted by the claims.

## Claims

1. A method in radiology, **characterised in that** as information relating to radiology at least image information of the object of the radiological procedure is projected to be visible in the field of vision of a person performing the radiological procedure, which information said person utilises when performing the radiological procedure.

2. A method according to claim 1, **characterised in that** the method in radiology is a mammography method, and that X-ray image information of the breast is arranged to be visible on the surface of the breast.

3. A method according to claim 1, **characterised in that** at least one control mark is arranged to be visible on the surface of the object, so that positioning of an instrument used in the radiological procedure is made easier and more accurate.

4. A method according to claim 1, **characterised in that** at least one imaging parameter used in the radiological procedure is arranged to be visible on the surface of the object or on a part of the radiological equipment arrangement.

5. A method according to claim 1, **characterised in that** in the method the view to the patient's visual organs is arranged during the radiological procedure so that the patient's visual organs see the red colour of blood as a colour different from red.

6. A method according to claim 1, **characterised in that** the information is arranged to be visible as green coloured light.

7. An equipment arrangement in radiology, **characterised in that** the equipment arrangement comprises a projector to project as information relating to radiology at least image information of the object of the radiological procedure to be visible in the field of vision of a person performing the radiological procedure, which information said person utilises when performing the radiological procedure.

8. An equipment arrangement in radiology according to claim 7, **characterised in that** the equipment arrangement in radiology is a mammography equipment arrangement which comprises means for arranging X-ray image information of the breast to be visible on the surface of the breast.

9. An equipment arrangement in radiology according to claim 7, **characterised in that** the arrangement in radiology comprises means to arrange at least one control mark to be visible on the surface of the object, so that positioning of an instrument used in the radiological procedure is made easier and more accurate.

10. An equipment arrangement in radiology according to claim 7, **characterised in that** the arrangement in radiology comprises means to arrange at least one imaging parameter used in the procedure to be visible on the surface of the object or on a part of the equipment arrangement.

11. An equipment arrangement in radiology according to claim 7, **characterised in that** the equipment arrangement comprises means to arrange a view to the patient's visual organs during the radiological procedure so that the patient's visual organs see the red colour of blood as a colour different from red.

12. An equipment arrangement in radiology according to claim 7, **characterised in that** the equipment arrangement comprises means for arranging information to be visible as green coloured light.
